# NOUVEAU FASCICULE DE BREVET EUROPEEN

(11) **EP 0 979 807 B2**
(45) Date de publication et mention de la décision concernant l'opposition: **21.09.2005**
(45) Mention de la délivrance du brevet: 14.11.2001
(21) Numéro de dépôt: 99401926.3
(22) Date de dépôt: 27.07.1999
(51) Int. Cl.: C07C 1/04, B01J 23/75

(54) **Procédé de synthèse Fischer-Tropsch en présence d'un catalyseur dont les particules métalliques ont une taille contrôlée**
Verfahren zur Fischer-Tropsch-Synthese in Gegenwart eines Katalysators mit vorbestimmter Grösse der Metallpartikel
Process for the Fischer-Tropsch synthesis in presence of a catalyst with controlled size of the metallic particles

(30) Priorité: 12.08.1998 FR 9810346
(43) Date de publication de la demande: 16.02.2000
(73) Titulaire: Institut Français du Pétrole, 92500 Rueil Malmaison (FR); ENI S.p.A., 00144 Roma (IT)
(72) Inventeur: Roy, Magalie, 92500 Rueil Malmaison (FR); Didillon, Blaise, 92500 Rueil Malmaison (FR); Uzio, Denis, 78160 Marly le Roi (FR); Verdon, Catherine, 78160 Marly le Roi (FR)
(74) Mandataire: Hartmann, Günter

(56) Documents cités:
- EP-A- 0 313 375
- EP-A- 0 466 984
- EP-A- 0 581 619
- EP-A- 0 736 326
- US-A- 4 626 552
- US-A- 4 681 867
- US-A- 4 714 692
- US-A- 4 714 693
- US-A- 4 945 116
- JOURNAL OF CATALYSIS, 1980, 65, p. 328-334
- "Stucture sensitivity of hydrocarbon sythesis from CO and H2" Boudart and McDonald, J. Phys. Chem. 1984, 88, p. 2185-2195
- "The Fischer-Tropsch Sythesis in the Liquid Phase" Kolbel and Ralek;Catal. Rev.-Sci. Eng., 1980, 21(2), p. 225-274
- "The Statoil GMD Slurry Process", Kinnari, Roterud, Rytter, Solbakken; AICE 1990 Spring National Meeting, March 18-22, 1990

## Description

La présente invention concerne le domaine des catalyseurs utilisés pour les réactions de synthèse d'hydrocarbures à partir de mélange de gaz comprenant du monoxyde de carbone et de l'hydrogène, généralement appelée synthèse Fischer-Tropsch.

Ainsi, la présente invention concerne l'utilisation d'un catalyseur comprenant au moins un support et au moins un métal du groupe VIII dans un procédé de synthèse d'hydrocarbures à partir d'un mélange comprenant du monoxyde de carbone et de l'hydrogène et éventuellement du dioxyde de carbone noté CO-(CO₂)-H₂₋

### Description de l'art antérieur

Les propriétés des catalyseurs métalliques supportés, c'est-à-dire des catalyseurs comprenant un métal ou plusieurs métaux déposé(s) sur un support choisi parmi les oxydes réfractaires, le charbon, les polymères ou tout autre matériau, sont conditionnées par un ensemble de paramètres, comme par exemple la taille des particules métalliques.

Il existe un grand nombre d'exemples dans la littérature démontrant l'influence de la taille des cristallites métalliques sur l'activité du catalyseur final. Cet aspect a été largement détaillé dans « Catalysis by Metals and Alloys », V. Pnec, E. Bond, Study in Surface Science and Catalysis, Volume 95, page 280, 1995. D'autre part, la diminution de la taille de ces cristallites est souvent liée à l'augmentation de l'interaction entre le métal et le support .

Les catalyseurs utilisés pour la conversion du gaz de synthèse en hydrocarbures, opérée à haute température et sous pression (connue dans la littérature sous le nom de synthèse Fischer-Tropsch) requièrent des conditions spécifiques de taille des cristallites.

Ainsi des métaux du groupe VIII de la classification périodique des éléments, tels que le fer, le ruthénium, le cobalt, le nickel catalysent la transformation de mélanges CO-(CO₂)-H₂ (c'est à dire les mélanges CO-H₂ comprenant éventuellement du CO₂, appelés gaz de synthèse) en hydrocarbures liquides et/ou gazeux.

Pour ces réactions, il est nécessaire de disposer de catalyseurs dont la taille des particules métalliques est contrôlée, c'est-à-dire que la taille des particules et la répartition des particules en fonction de la taille n'est pas aléatoire, afin d'optimiser l'activité et la sélectivité du catalyseur.

Les méthodes conventionnelles de préparation des catalyseurs métalliques supportés utilisés pour la synthèse Fischer-Tropsch consistent à déposer un sel métallique ou un complexe de coordination métal-ligand sur le support, puis à réaliser une étape d'activation consistant en un ou plusieurs traitement(s) thermique(s) réalisés sous air et/ou sous hydrogène, ce qui entraîne une mauvaise répartition de la taille des particules.

La demande de brevet EP 0736326 A décrit une nouvelle méthode de préparation de catalyseur au cobalt obtenu par séchage à une pression inférieure à la pression atmosphérique : les tailles des particules de cobalt obtenues sont comprises entre 10 et 17 nm.

La demande de brevet EP 0174696 A décrit un procédé de préparation d'un catalyseur Fischer-Tropsch à base de cobalt, où le cobalt est réparti de telle façon que ΣVp/Σ Vc < 0.85 (ΣVp étant la quantité de Co contenu dans la périphérie du solide. ΣVc étant la quantité de Co contenu dans la totalité du solide). L'inventeur montre que cette répartition favorise la formation de C₅₊ Le catalyseur est préparé par imprégnation du support (préférentiellement la silice) déjà immergé dans l'eau pendant 30 secondes et peut contenir un promoteur, de préférence le zirconium.

Le brevet US 4,977,126 décrit également une méthode de dispersion en surface par vaporisation d'un liquide dans lequel le composé métallique est dissous. Une couche périphérique est ainsi formée.

D'autres brevets US 4,605,679 et US 4,729,981 et la demande de brevet EP 0535790A décrivent des modifications de l'étape de réduction / activation

Par ces différentes techniques, la distribution en taille des particules de métal du groupe VIII et leur interaction avec le support ne sont cependant pas bien maîtrisées.

Il est donc avantageux, afin d'améliorer l'activité des catalyseurs en utilisation dans un procédé de synthèse d'hydrocarbures, de disposer de catalyseurs métalliques supportés pour lesquels la taille moyenne et leur distribution en taille est maîtrisée et les interactions avec le support limitées, grâce à une nouvelle méthode de préparation. En effet, il a été démontré dans la littérature que l'interaction entre le métal ou les métaux constituant le catalyseur et le support utilisé conditionne la réductibilité et l'activité du catalyseur (cf. Z. Karpinski, Adv. Catal., Vol.37,p.45, 1990).

Cette interaction entre le métal et le support peut être définie par un ensemble de techniques de caractérisation connues de l'homme du métier. On peut par exemple citer la thermoréduction programmée qui consiste à déterminer la température de réduction de l'oxyde supporté en métal. Il a en effet été mis en évidence que plus la température de réduction de l'oxyde supporté en métal est élevée plus l'interaction entre le métal et le support est élevée.

### Résumé de l'invention

Il a été découvert, et ceci fait l'objet de la présente invention, un nouveau procédé de synthèse d'hydrocarbures à partir d'un mélange de gaz de synthèse comprenant du monoxyde de carbone et de l'hydrogène et éventuellement du dioxyde de carbone en présence d'un catalyseur comprenant un support, au moins un métal du groupe VIII, c'est-à-dire les groupes 8, 9 et 10 de la nouvelle classification périodique (Handbook of Chemistry and Physics, 76^{ème} édition, 1995-1996, intérieur de la page de couverture), ledit procédé étant caractérisé comme indiqué dans la revendication 1.

Les particules métalliques du catalyseur ont généralement une taille moyenne supérieure à 1 nm.

Le procédé selon l'invention est alors effectué en présence d'un catalyseur ayant une réductibilité améliorée, c'est à dire que la température nécessaire à la réduction du catalyseur est inférieure à la température nécessaire à la réduction des catalyseurs de l'art antérieur. En effet le catalyseur utilisé dans le procédé de synthèse d'hydrocarbures selon l'invention présente une diminution de l'interaction entre le métal du groupe VIII et le support par rapport aux catalyseurs de l'art antérieur, ce qui permet une augmentation de l'activité.

### Description détaillée de l'invention

L'invention concerne donc l'utilisation d'un catalyseur dans un procédé de synthèse d'hydrocarbures. Le catalyseur est caractérisé en ce qu'au moins 80 % des particules d'au moins un métal du groupe VIII ont une taille comprise entre D-(D.0,2) et D+(D.0.2), où D représente la taille moyenne des particules.

Le catalyseur est synthétisé par la préparation d'une suspension colloïdale en phase aqueuse d'au moins un oxyde du métal ou des métaux du groupe VIII à supporter, suivie du dépôt de ladite suspension sur un support, suivi de la réduction éventuelle du ou des oxydes ainsi supportés.

Dans la méthode de préparation du catalyseur utilisé dans le procédé selon l'invention, la suspension colloïdale d'au moins un oxyde métallique d'un métal du groupe VIII est préparée en phase aqueuse. Par rapport à d'autres méthodes de préparation de suspension colloïdales, cette méthode utilise l'eau comme solvant, ce qui présente un avantage au plan de la sécurité, et conduit à des économies en terme de coût du procédé.

La méthode de préparation du catalyseur utilisé dans le procédé selon la présente invention comprend plusieurs étapes :
a) préparation d'une suspension colloïdale d'au moins un oxyde métallique, en phase aqueuse
b) dépôt de la suspension colloïdale par imprégnation sur un support ;
c) séchage à une température inférieure ou égale à 250°C
d) calcination éventuelle à une température pouvant aller jusqu'à 700°C
e) réduction

Dans l'étape a) la suspension colloïdale est obtenue par hydrolyse d'au moins un cation métallique d'au moins un métal du groupe VIII en milieu aqueux, ce qui conduit à la formation de particules d'oxydes ou d'hydroxydes en suspension.

L'hydrolyse est réalisée par neutralisation d'une solution aqueuse contenant un sel précurseur du métal du groupe VIII susceptible de conduire à l'hydroxyde métallique ou à l'oxyde métallique, la neutralisation est effectuée au moyen d'au moins une base minérale telle que par exemple la soude, la potasse ou une solution d'ammoniaque.

Le métal du groupe VIII est de préférence choisi parmi le cobalt, le fer, le ruthénium et le nickel, et de manière plus préférée le cobalt.

Les sels de métal du groupe VIII utilisables dans la méthode de préparation selon l'invention sont de préférence les nitrates, ou tout autre sel susceptible de générer un précurseur du métal du groupe VIII en solution et pouvant conduire à des hydroxydes ou oxydes qui donnent lieu à la formation de particules en suspension.

La solution contenant la base minérale utilisée pour la neutralisation peut être par exemple versée dans la solution aqueuse contenant le ou les sels métalliques ou, inversement, on peut verser la solution contenant le ou les sels métalliques dans la solution contenant la base minérale. On peut aussi préparer la suspension colloïdale en versant au moins en partie simultanément les deux solutions dans l'appareillage servant à préparer la suspension colloïdale.

Lors de la préparation de la suspension colloïdale d'oxydes métalliques, un ou plusieurs autres sels métalliques peuvent éventuellement être introduits à tout instant de la préparation. Ce ou ces sels peuvent éventuellement conduire à la formation d'oxyde(s) ou d'hydroxyde(s) en milieux aqueux ou ne pas être transformés dans le milieu réactionnel. Ces métaux additionnels sont de préférence le ruthénium, le tantale et le molybdène.

Des composés visant à stabiliser la suspension colloïdale peuvent être introduits lors de la préparation de la suspension colloïdale. Parmi ces composés, on peut par exemple citer les protons hydrogène, les nitrites, l'éthylènediamine sans que cette liste soit limitative.

Après hydrolyse réalisée par neutralisation d'au moins une partie de la solution comprenant les sels de métal du groupe VIII, il est éventuellement possible de laisser la suspension colloïdale sous agitation, éventuellement après modification du pH par ajout de quantités d'acide ou de base compatibles avec la stabilité de la suspension colloïdale.

Dans le cas où le catalyseur doit être débarrassé de la majeure partie des métaux alcalins ou alcalino-terreux qu'il peut contenir, il est possible d'éliminer la majorité desdits métaux par ajout à la suspension colloïdale d'une solution aqueuse au pH ajusté. La suspension ou le précipité obtenu est ensuite filtré et lavé au moyen d'une solution aqueuse, puis, si nécessaire, est remis en suspension dans une autre solution aqueuse dont le pH et la composition sont contrôlés afin d'obtenir à nouveau une suspension colloïdale. Cette suspension peut ensuite être imprégnée sur le support. Ces opérations peuvent éventuellement être effectuées en continu.

Lors de la préparation de la suspension colloïdale, le contrôle des paramètres opératoires tels que le pH, le temps d'addition des composés formant la solution colloïdale, la température, la durée de maturation, l'ajout d'additifs à différents moments de la préparation, la concentration du milieu ou la force ionique, permet de contrôler la taille des particules d'oxyde dans le milieu, leur nombre et leur état d'agrégation.

En général, la température de préparation est comprise entre -10°C et 100°C, de préférence entre 0°C et 50°C, et de manière très préférée entre 0°C et 35°C. La durée de maturation peut généralement varier entre 0 et 40 heures, de préférence entre 15 minutes et 24 heures, et la force ionique est de préférence comprise entre 0,005 et 20 moles par litre, de manière plus préférée entre 0,01 et 10 moles par litre.

Dans l'étape b) la suspension colloïdale contenant le métal ou les métaux sous formes de particules d'oxydes ou d'hydroxydes est ensuite imprégnée sur un support. Le support peut être choisi parmi tous les supports déjà décrits dans la littérature. On peut par exemple utiliser au moins un composé choisi parmi les oxydes réfractaires, tels que la silice, l'alumine, la magnésie, les silice-alumines, les silicoaluminates, les charbons, les supports organiques.

Le support peut éventuellement subir un ensemble de traitements avant l'étape d'imprégnation tels que des calcination, des hydratations... Le support, peut aussi déjà contenir un ou plusieurs éléments métalliques soit introduit (s) avant l'imprégnation de la suspension colloïdale par des techniques conventionnelles, soit introduit(s) lors de la synthèse du catalyseur utilisé dans le procédé selon l'invention.

L'imprégnation est de préférence réalisée dans des conditions telles que le volume de solution correspond environ au volume poreux du support. D'une façon préférée, la suspension colloïdale est versée sur le support. Ce processus peut être réalisé de façon discontinue, c'est-à-dire que l'étape de préparation de la suspension colloïdale précède l'imprégnation de ladite solution sur le support, ou de façon continue.

On peut par exemple décrire comme processus continu un processus ou l'hydrolyse d'une solution contenant au moins un sel métallique est réalisée par neutralisation au moyen d'au moins une base minérale. Par exemple les deux solutions sont versées simultanément dans un bac qui, par trop plein, se déverse dans une zone contenant le support de catalyseur.

Après imprégnation, le catalyseur est séché dans l'étape c) à une température inférieure ou égale à 250°C afin d'éliminer tout ou une partie de l'eau introduite lors de l'imprégnation.

Après séchage, le catalyseur peut éventuellement être calciné dans l'étape d) à une température allant jusqu'à 700°C.

Ensuite le catalyseur est réduit dans l'étape e). La réduction de l'oxyde de métal du groupe VIII est réalisée au moyen de tout composé réducteur, par exemple l'hydrogène moléculaire ou le monoxyde de carbone ou un mélange des deux. La réduction peut avoir lieu soit in-situ, c'est-à-dire dans le réacteur où est réalisée la transformation catalytique, soit ex-situ, c'est-à-dire dans un outil différent de celui où a lieu la réaction catalytique, soit en partie in-situ et en partie ex-situ. Cette pré-réduction peut être réalisée en phase gazeuse ou dans une phase liquide comprenant au moins un hydrocarbure ayant au moins 5, de préférence au moins 10, atomes de carbone par molécule si, par la suite la réaction de synthèse d'hydrocarbures se déroule dans une phase liquide comprenant au moins un hydrocarbure ayant au moins 5, de préférence au moins 10 atomes de carbone par molécules.

Le procédé selon l'invention concerne l'utilisation des catalyseurs tels que décrits ci-dessus dans les procédés de fabrication d'un mélange d'hydrocarbures essentiellement linéaires et saturés, contenant au moins 25 % en poids d'hydrocarbures C₅₊ par rapport à l'ensemble des hydrocarbures formés, à partir d'un gaz de synthèse.

Les conditions de mise en oeuvre du procédé pour la fabrication d'hydrocarbures selon l'invention sont habituellement les suivantes : La conversion du gaz de synthèse est opérée sous une pression totale comprise entre 0,1 et 15 MPa, et de préférence entre 1 et 10 MPa, la température est généralement comprise entre 150 et 350°C, et de préférence entre 170 et 300°C. La vitesse volumique horaire est habituellement comprise entre 100 et 20 000 volumes de gaz de synthèse par volume de catalyseur et par heure et de préférence entre 400 et 10 000 volumes de gaz des synthèse par volume de catalyseur et par heure , et le rapport H₂/CO dans le gaz de synthèse est habituellement compris entre 1 : 2 et 5 : 1 ; de préférence entre 1,2 : 1 et 2,5 : 1.

Le procédé selon l'invention peut être mis en oeuvre dans tout type de réacteur. On utilisera de préférence un réacteur à lit fixe de catalyseur ou un réacteur opérant en phase liquide tel un réacteur dans le lequel le catalyseur est en suspension dans la phase liquide (slurry) ou tel un réacteur opérant avec un lit ruisselant de catalyseur (trickle bed).

Les exemples qui suivent permettent d'illustrer l'invention sans en limiter la portée.

### Exemple 1 (selon l'invention):

Un catalyseur A est préparé par imprégnation d'une solution colloïdale d'oxyde de cobalt sur une poudre d'alumine de surface spécifique égale à 180 m²/g. La suspension colloïdale est obtenue en ajoutant une solution d'ammoniaque (4 mol/l) à une solution de nitrate de cobalt (50 g/l) pour atteindre un pH = 8.

Le catalyseur est séché à 120°C et calciné à 250°C. La teneur finale en cobalt est de 8%. La taille moyenne des particules de cobalt obtenu par DRX (Diffraction aux rayons X) est de 15 nm. L'analyse microscopique montre que selon l'invention, plus de 80% des particules ont une taille comprise entre 12,5 et 17,5 nm.

La courbe TPR montre que le catalyseur est totalement réduit à une température inférieure à 300°C sous un mélange H₂/Ar contenant 5% d'hydrogène.

### Exemple 2 (comparatif) :

Un catalyseur B est préparé à partir du même support que le catalyseur A par imprégnation à sec d'une solution de nitrate de cobalt. Le catalyseur est séché à 120°C, puis calciné à 400°C de manière à décomposer le nitrate de cobalt en oxyde. La teneur finale en cobalt est de 11,5%. L'analyse DRX montre la présence de particules de cobalt de taille comprise entre 15 et 30 nm, les tailles de particules étant réparties de façon aléatoire.

La courbe TPR montre que sous un mélange H₂/Ar contenant 5% d'hydrogène il faut monter à une température supérieure à 600°C pour réduire totalement l'oxyde de cobalt.

### Exemple 3 (selon l'invention)

Un catalyseur C est préparé par imprégnation d'une solution colloïdale d'oxyde de cobalt sur une poudre d'alumine de surface spécifique égale à 180 m²/g. La suspension colloïdale est obtenue en ajoutant un solution de potasse 0.75N à une solution de nitrate de cobalt (50g/l) pour atteindre un pH égal à 7.5.

Le catalyseur est ensuite lavé sur Büchner de manière à éliminer les ions potassium.

La catalyseur est ensuite séché à 120°C et calciné à 250°C.

La teneur finale en cobalt est de 6%. L'analyse DRX montre la présence de particules de cobalt de taille moyenne égale à 10 nm. L'analyse microscopique montre que selon l'invention, plus de 80% des particules ont une taille comprise entre 8 et 12 nm

La courbe TPR montre que le catalyseur est totalement réduit à une température inférieure à 300°C sous un mélange H₂/Ar contenant 5% d'hydrogène.

### Exemple 4 : évaluation des performances des catalyseurs A, B, C

Les catalyseurs A, B, C dont les préparations sont décrites dans les exemples ci-dessus ont été testés dans la réaction de conversion du gaz de synthèse (CO-H₂). La réaction a lieu en lit fixe en phase gazeuse dans une unité fonctionnant en continu et opérant sur 20 cm³ de catalyseur. Les catalyseurs sont préalablement réduits «in situ» sous hydrogène pur à pression atmosphérique, à 250°C pour les catalyseurs selon l'invention A et C, à 350°C pour le catalyseur B. Les conditions de test sont les suivantes :
T = 220°C
P=2 M Pa
vitesse volumique horaire: VVH = 1500 h⁻¹
rapport molaire H₂/CO = 2

Les résultats sont les suivants:

| Catalyseur | % Co (poids) | Conversion du CO (%) (par g de Co) | Distribution des produits formés (% poids) | | |
|---|---|---|---|---|---|
| | | | C₁ | C₁-C₄ | C₅₊ |
| A | 8 | 28 | 10 | 22 | 78 |
| B | 12 | 25 | 13 | 25.5 | 74.5 |
| C | 6 | 20 | 12 | 23 | 77 |

Ces résultats montrent que l'utilisation des catalyseurs dans un procédé de conversion du gaz de synthèse permet un gain quant à la température de réduction nécessaire pour réduire les oxydes de cobalt en cobalt d'au moins 100°C par rapport au procédé en présence d'un catalyseur selon l'art antérieur, tout en présentant des performances catalytiques aussi bonnes voire meilleures que les procédés de l'art antérieur.

## Revendications

1. Procédé de synthèse d'hydrocarbures à partir d'un mélange comprenant du monoxyde de carbone et de l'hydrogène en présence d'un catalyseur comprenant un support et au moins un métal du groupe VIII,
ledit procédé étant **caractérisé en ce que** ledit catalyseur est préparé par
a) préparation d'une suspension colloidale d'au moins un oxyde d'un métal du group VIII en phase aqueuse par hydrolyse d'au moins un cation métallique d'au moins un métal du groupe VIII en phase aqueuse au moyen d'au moins une base minérale;
b) dépôt de la suspension colloïdale par imprégnation sur ledit support;
c) séchage à une température inférieure ou égale à 250 °C;
d) éventuellement calcination à une température pouvant aller jusqu' à 700 °C; et
e) réduction,
de manière à ce qu'au moins 80 % des particules métalliques du groupe VIII du catalyseur ont une taille comprise entre D-(D.0,2) et D+(D.0,2), où D représente la taille moyenne des particules metalliques et ledit catalyseur est utilisé pour la synthèse d'hydrocarbures.

2. Procédé selon la revendication 1 tel que le mélange comprend en outre du dioxyde de carbone.

3. Procédé selon la revendication 1 ou 2 tel que la base minérale utilisé pour la préparation du catalyseur, est choisie parmi la soude, la potasse, une solution d'ammoniaque.

4. Procédé selon l'une des revendications 1 à 3 tel que le métal du groupe VIII du catalyseur utilisé est choisi parmi le cobalt, le fer, le ruthénium et le nickel.

5. Procédé selon la revendication 4 tel que le métal du groupe VIII est le cobalt.

6. Procédé selon l'une des revendications 1 à 5 tel que avant le dépôt de la suspension colloidale sur le support au moins un composé d'au moins un élément choisi parmi le ruthénium, le tantale, le molybdène est ajouté à la solution colloidale.

7. Procédé selon l'une des revendications 1 à 6 tel que le catalyseur utilisé a été préparé à une température comprise entre -10°C et 100°C, pendant une durée de maturation comprise entre 0 et 40 heures, et avec une force ionique comprise entre 0,005 et 20 moles par litre.

8. Procédé selon l'une des revendications 1 à 7, tel qu'il est opéré sous une pression totale comprise entre 0,1 et 15 MPa, à une température comprise entre 150 et 350°C, avec une vitesse volumique horaire comprise entre 100 et 20 000 volumes de gaz de synthèse par volume de catalyseur et par heure, le rapport H₂/CO dans le gaz de synthèse étant compris entre 1 : 2 et 5 : 1.

9. Procédé selon l'une des revendications 1 à 8 tel qu'il est opéré dans un réacteur à lit fixe de catalyseur.

10. Procédé selon l'une des revendications 1 à 8 tel qu'il est opéré dans un réacteur fonctionnant en phase liquide dans lequel le catalyseur est en suspension dans la phase liquide.

## Patentansprüche

1. Verfahren zur Synthese von Kohlenwasserstoffen, ausgehend von einer Mischung, die Kohlenmonoxid und Wasserstoff umfasst, in Gegenwart eines Katalysators, der einen Träger und wenigstens ein Metall der Gruppe VIII umfasst, wobei das Verfahren **dadurch gekennzeichnet ist, dass** der Katalysator hergestellt wird durch
a) Herstellung einer kolloidalen Suspension wenigstens eines Oxids eines Metalls der Gruppe VIII in wässriger Phase durch Hydrolyse wenigstens eines Metallkations wenigstens eines Metalls der Gruppe VIII in wässriger Phase mittels wenigstens einer mineralischen Base,
b) Abscheidung der kolloidalen Suspension durch Imprägnierung auf einem Träger,
c) Trocknung bei einer Temperatur von ≤ 250 °C,
d) gegebenenfalls Calcinierung bei einer Temperatur, die bis zu 700 °C betragen kann, und
e) Reduktion,
in der Weise, dass wenigstens 80 % der Teilchen des Metalls der Gruppe VIII des Katalysators eine Größe haben, die zwischen D-(D.0,2) und D+(D.0,2) liegt, wobei D die mittlere Größe der Metallteilchen darstellt, und dass der Katalysator für die Synthese von Kohlenwasserstoffen verwendet wird.

2. Verfahren nach Anspruch 1, derart, dass die Mischung außerdem Kohlendioxid umfasst.

3. Verfahren nach Anspruch 1 oder 2, derart, dass die für die Herstellung des Katalysators verwendete mineralische Base unter Soda, Pottasche und einer Ammoniak-Lösung ausgewählt ist.

4. Verfahren nach einem der Ansprüche 1 bis 3, derart, dass das Metall der Gruppe VIII des Katalysators aus Kobalt, Eisen, Ruthenium und Nickel ausgewählt ist.

5. Verfahren nach Anspruch 4, derart, dass das Metall der Gruppe VIII Kobalt ist.

6. Verfahren nach einem der Ansprüche 1 bis 5, derart, dass vor der Abscheidung der kolloidalen Suspension auf dem Träger wenigstens eine Verbindung wenigstens eines Elements, ausgewählt aus Ruthenium, Tantal und Molybdän, zu der kolloidalen Lösung zugegeben wird.

7. Verfahren nach einem der Ansprüche 1 bis 6, derart, dass der verwendete Katalysator bei einer Temperatur zwischen -10 °C und 100 °C, während einer Reifedauer, die zwischen 0 und 40 h liegt, und bei einer lonenstärke, die zwischen 0,005 und 20 mol pro Liter liegt, hergestellt worden ist.

8. Verfahren nach einem der Ansprüche 1 bis 7, derart, dass es unter einem Gesamtdruck zwischen 0,1 und 15 MPa bei einer Temperatur zwischen 150 und 350 °C mit einer stündlichen Raumgeschwindigkeit zwischen 100 und 20 000 Volumina Synthesegas pro Volumen Katalysator und pro Stunde durchgeführt wird, wobei das Verhältnis H₂/CO in dem Synthesegase zwischen 1 : 2 und 5 : 1 liegt.

9. Verfahren nach einem der Ansprüche 1 bis 8, derart, dass es in einem Reaktor mit einem Katalysator-Fixbett durchgeführt wird.

10. Verfahren nach einem der Ansprüche 1 bis 8, derart, dass es in einem Reaktor durchgeführt wird, der in flüssiger Phase arbeitet, und in dem der Katalysator in der flüssigen Phase in Suspension vorliegt.

## Claims

1. A process for the synthesis of hydrocarbons starting from a mixture containing carbon monoxide and hydrogen in the presence of a catalyst comprising a support and at least one group VIII metal, said process being **characterized in that** the catalyst is prepared by
a) preparation of a colloidal suspension of at least one oxide of a group VIII metal in aqueous phase by the hydrolysis of at least one metallic cation of at least one group VIII metal in aqueous phase by using at least one mineral base,
b) deposition of the colloidal suspension by impregnation onto a support,
c) drying at a temperature lower than or equal to 250°C,
d) optional calcination at a temperature which may be up to 700 °C, and
e) reduction
in such manner that at least 80% of the group VIII metallic particles of the catalyst have a size comprised between D-(D.0.2) and D+(D.0.2), where D represents the average size of the metallic particles, and that said catalyst is used for the synthesis of hydrocarbons.

2. The process according to claim 1 such that the mixture contains in addition carbon dioxide.

3. The process according to claim 1 or 2 such that the mineral base used for the preparation of the catalyst is chosen from soda, potash, and an ammonia solution.

4. The process according to one of claims 1 to 3 such that the group VIII metal of the catalyst is chosen from cobalt, iron, ruthenium and nickel.

5. The process according to claim 4 such that the group VIII metal is cobalt.

6. The process according to one of claims 1 to 5 such that before depositing the colloidal suspension on the support at least one compound of at least one element chosen from the group constituted by ruthenium, tantalum and molybdenum is added to the colloidal solution.

7. The process according to one of claims 1 to 6 such that the used catalyst has been prepared at a temperature comprised between -10°C and 100°C, for a duration of maturation comprised between 0 and 40 hours, and with an ionic strength comprised between 0.005 and 20 moles per liter.

8. The process according to one of claims 1 to 7 such that it is operated under a total pressure comprised between 0.1 and 15 MPa, at a temperature comprised between 150 and 350°C with a hourly volume velocity comprised between 100 and 20, 000 volumes of synthesis gas per volume of catalyst and per hour, the H₂/CO ratio in the synthesis gas being comprised between 1:2 and 5:1.

9. The process according to one of claims 1 to 8 such that it is operated in a catalyst fixed-bed reactor.

10. The process according to one of claims 1 to 8 such that it is operated in a reactor working in liquid phase wherein the catalyst is suspended in the liquid phase.
